# EUROPEAN PATENT SPECIFICATION

(11) **EP 1 541 183 B1**
(45) Date of publication and mention of the grant of the patent: **12.08.2009**
(21) Application number: 04257324.6
(22) Date of filing: 25.11.2004
(51) Int. Cl.: A61L 31/14, A61F 2/02

(54) **Prosthetic repair device**
Prosthetische Reparaturvorrichtung
Dispositif de réparation prosthétique

(30) Priority: 26.11.2003 US 723720
(43) Date of publication of application: 15.06.2005
(73) Proprietor: ETHICON, INC., Somerville, NJ 08876-0151 (US)
(72) Inventor: Popadiuk, Nicholas, Hillsborough, NJ 08844 (US); Egidio, Dominick, Flanders, New Jersey 07836 (US); Keilman, Kenneth, Raritan, New Jersey 08869 (US)
(74) Representative: Mercer, Christopher Paul

(56) References cited:
- EP-A- 0 797 962
- EP-A- 1 099 422
- EP-A- 1 306 061
- WO-A-03/041613
- US-A- 5 593 441
- US-A- 5 686 090
- US-A1- 2003 040 809
- US-B1- 6 319 264

## Description

### BACKGROUND OF THE INVENTION

A large defect in the abdominal wall, not amenable to primary closure, may require insertion of a prosthesis to close and repair the defect. Typically, for a period of 3 to 6 months following the repair procedure, the site of the defect gradually builds up scar tissue, which strengthens the site. The ideal prosthesis is incorporated by surrounding tissue, does not stimulate adhesions, and has appropriate strength and pliability.

Prostheses having a nonabsorbable porous material and an absorbable anti-adhesion material are well known in the prior art. For example, a prosthetic repair device having a polypropylene mesh such as Marlex® mesh and a gelatin film such as Gelfilm® absorbable film is described by Jenkins et al., in "A Comparison of Prosthetic Materials Used to Repair Abdominal Wall Defects", Surgery, Vol. 94, No. 2, August 1983, pg. 392-398.

USP 5,593,441 to Lichtenstein et al describes a prosthetic repair device preferably having a sheet of polypropylene mesh that allows tissue in-growth, such as Marlex® mesh, and an adhesion barrier. Although the adhesion barrier described by Lichtenstein et al is preferably a sheet of silicone elastomer, Lichtenstein et al suggest that that an oxidized regenerated cellulose such as Interceed® (TC7) absorbable adhesion barrier (commercially available from Ethicon, Inc., in Somerville, New Jersey) having only short term effectiveness may be used as the adhesion barrier.

USP 5,686,090 to Schilder et al describes the use of a fleece in combination with a woven or knit mesh to control the speed of tissue proliferation into the mesh. Schilder et al also suggest the use of a nonabsorbable or absorbable film to prevent mis-growths to adjacent tissue and to reduce adhesions.

US 2003/0040809 to Goldmann et al describes a fabric having two sides, where one side has a three-dimensional microstructure permitting an in-growth of cells and the other a substantially closed surface that is unfavorable for the adhesion of cells. This reference teaches that the three-dimensional microstructure is formed from polypropylene, polyester, or polytetrafluoroethylene, or polyylactides, polyglycolides and copolymers thereof if resorbability or partial resorbability is desired. Goldmann et al also describe the substantially closed surface as being made from polyurethane, or polylactides and copolymers thereof if resorbability or partial resorbability is desired, and that additional adhesion prevention may be provided by using a bioabsorbable component such as a polymer or copolymer of organic hydroxyesters, polyglycolide, polylactide, polydioxanone, polyhydroxy butyric acid, polycaprolactone, polytrimethylene carbonate and polyvinyl alcohol, to provide a sealing effect on the outer surface of the substantially closed surface.

In contrast to the conventional view of using a porous side to support tissue in-growth as described above, WO 03/0416131 describes prostheses having a mesh structure that is provided on both sides with a film to form an adhesion barrier on both sides of the mesh. Specifically, WO 03/0416131 teaches that two polymer films are glued or welded together in the pores of the mesh. Preferred materials for the mesh are polypropylene and mixtures of polyvinylidene fluoride and copolymers of vinylidene fluoride and hexafluoropropene. This reference teaches the use of a polymer film, such as poly-p-dioxanone, on each side of the mesh.

EP-A-1 306 061 describes a prosthetic repair device comprising a nonabsorbable mesh encapsulated with an absorbable material. Additionally, an absorbable spring is encapsulated with the absorbable material.

US 6,319,264 discloses a prosthetic repair device comprising a first, rapid absorbable layer and a second, more slowly absorbable layer.

Although prior art references teach and suggest prostheses having nonabsorbable porous materials and absorbable adhesion barriers, there remains difficulties in using the prior art prosthetic repair devices during minimally invasive laparoscopic surgery. For example, a hernia defect in the abdominal wall may be repaired via minimally invasive laparoscopic surgery, which is conducted through several small incisions through which the surgeon inserts trocars. During this type of surgery, the surgeon inserts instruments for making incisions and gripping tissue and surgical devices through the trocars. For example, using the instruments inserted into the trocars, the surgeon may first pull the hernial sac back into the abdominal cavity to expose the defect In the abdominal wall. The prosthetic repair device is also introduced to the site of the defect via the trocar and positioned to cover the defect with gripping instruments via the trocars. Difficulties encountered by the surgeon include difficulty in moving the prosthetic repair device through the trocar, unfurling the prosthetic repair device to a shape that can cover the defect, and correctly positioning the repair device to cover the defect. Therefore, it is desirable to have a prosthetic repair device that is simpler for the surgeon to use during laproscopic surgery, while exhibiting the properties of an ideal prosthetic repair device. For example, the ideal prosthetic repair device should be capable of being incorporated by surrounding tissue at a sufficient rate, does not stimulate adhesions, and has appropriate strength and pliability for the repair device.

### BRIEF SUMMARY OF THE INVENTION

According to the invention, there is provided a prosthetic repair device as set forth in the accompanying claim 1.

Further preferred aspects are set out in the dependent claims.

### BRIEF DESCRIPTION OF THE DRAWINGS

Figure 1 is a schematic perspective partial cut-away view of one embodiment of the prosthetic repair device.
Figure 2 is a schematic cross-sectional view of the embodiment of Figure 1 along the line 2-2.

### DETAILED DESCRIPTION OF THE INVENTION

The prosthetic repair devices described herein exhibit superior handling properties, combine the strength and pliability of, for example, a prosthetic mesh with the low incidence of postoperative adhesions of a physical barrier, while being capable of incorporation by surrounding tissue at a sufficient rate.

A first disclosed example, which is outside the scope of the claimed invention, is directed to a prosthetic repair device comprising a nonabsorbable material, a first absorbable material having a first absorption rate, and a second absorbable material having a faster absorption rate than the first absorption rate. In this example, the first absorbable material may function to isolate the nonabsorbable material from the internal or abdominal viscera or tissue and organs for a period of time after implantation, and/or as a means to join the nonabsorbable material to the second absorbable material of the prosthetic repair device when melted. Optionally, the first absorbable material may have a melting point that is lower than the melting points of either the nonabsorbable material or the second absorbable material. Additionally, one or more first absorbable material, second absorbable material or nonabsorbable material may be used in the repair device.

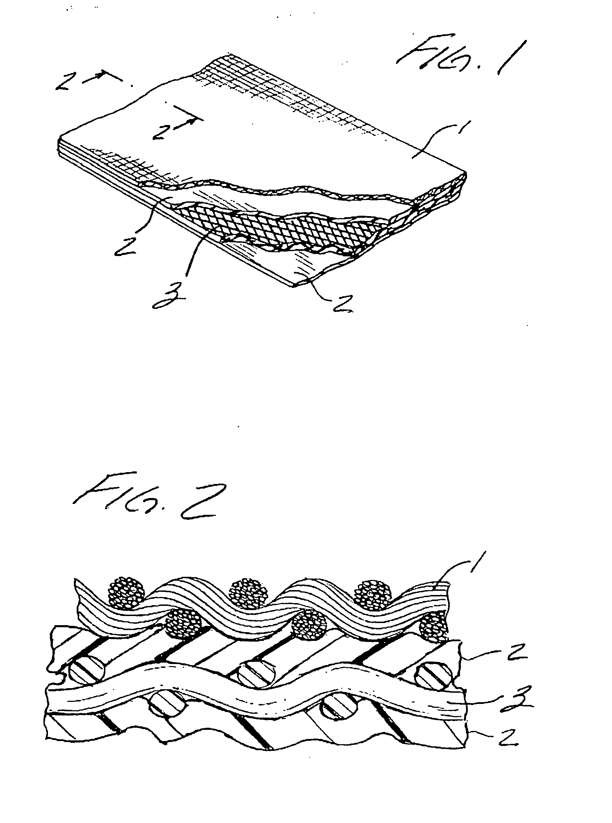

An embodiment according to the invention, as shown in Figures 1 and 2, is directed to a prosthetic repair device comprising a nonabsorbable porous material 3 that is encapsulated with a first absorbable component 2, and a second absorbable material 1 having a faster absorption rate than the first absorbable component. The first absorbable component in this embodiment may function to isolate the nonabsorbable material from the internal or abdominal viscera or tissue and organs for a period of time after implantation, and/or as a means to join the encapsulated nonabsorbable material and the second absorbable material of the prosthetic repair device when melted. Optionally, the first absorbable component may have a melting point that is lower than the melting points of either the nonabsorbable material or the second absorbable material. Additionally, one or more first absorbable component, second absorbable material or nonabsorbable material may be used in the repair device.

When the prosthetic repair device of the inventive embodiment is positioned for example intraperitoneally, the side of the encapsulated nonabsorbable material that is adjacent the peritoneum may releasably adhere to the peritoneum without requiring the surgeon to tack the prosthetic repair device in place prior to permanently affixing the device to the peritoneum. This is accomplished, for example, when there is sufficient surface adhesion between the first absorbable component on the side of the nonabsorbable material that is adjacent the peritoneum and the wet peritoneum itself. In this manner, the surgeon is able to position and/or reposition the prosthetic repair device over the defect multiple times until the device is in proper position. Further, since the surface adhesion between the first absorbable component on the side of the nonabsorbable material that is adjacent the peritoneum and the wet peritoneum holds the device in place, there is no need for the surgeon to use external forces, either manual or laproscopic, to hold the repair device in place, thereby freeing the surgeon's hands.

A simple parameter that may used to evaluate the ability of the prosthetic repair device to adhere to the abdominal wall is referred to herein as the hold time of the device.

In order to evaluate the hold time of the device, a 11.4 cm x 11.4 cm (4.5" x 4.5 ") chamois made from tanned sheepskin obtained from Acme Sponge & Chamois Co., Inc. and having a weight of 5 grams; and a 10.2 cm x 10.2 cm (4" x 4") block of any height and having a weight of 45 grams are utilized. The hold time of the device is measured at a temperature of 22.2°C (72°F), utilizing 10 cc of water placed in the center of a container that is large enough to accommodate at least one of the 10.2 cm x 10.2 cm (4" x 4") surfaces of the block. The 11.4 cm x 11.4 cm (4.5" x 4.5") chamois is wrapped to cover at least one of the 10.2 cm x 10.2 cm (4" x 4") surfaces of the block. The surface of the block covered with the chamois is then placed on top of the water in the container. Meanwhile, a 7.6 cm x 7.6 cm (3" x 3") dry sample of the prosthetic repair device to be evaluated is placed flat on a hard flat surface, with the side of the device that would be in contact, for example, with the abdominal wall during surgery facing upward. After the chamois has absorbed all the water in the container, the wet chamois and the block are removed from the container and gently dropped onto the upward side of the device, such that the weight of the block with the wet chamois is the only force applied to the device. The block and the wet chamois, with the device adhered thereto, is gently lifted from the flat surface and suspended in air until the device falls free of the chamois. The period of time beginning with when the block, wet chamois and device are lifted from the flat surface to the time the device falls free of the chamois is recorded as the hold time. In the event the device does not attach to the chamois, a hold time of zero is recorded.

For example, one embodiment of the prosthetic repair device described herein exhibits a hold time as long as 30 minutes. Preferably, the hold time of the prosthetic repair device ranges from 5 minutes to 30 minutes. More preferably, the hold time ranges from 10 to 20 minutes.

In the inventive embodiment where the first absorbable component encapsulates the nonabsorbable material, the thickness of the first absorbable component ranges from 0.1 to 1.2 mm on one side of the nonabsorbable material and from 0.1 to 1.2 mm on the other side, as measured from the planar surfaces of the nonabsorbable material.

Examples of the first absorbable material or component include but are not limited to polydioxanone such as poly(1,4-dioxan-2-one), polymers or copolymers of organic hydroxyesters, polyglycolide, polylactide, polyhydroxy butyric acid, polycaprolactone, polytrimethylene carbonate and polyvinyl alcohol.

The second absorbable material may function to isolate the nonabsorbable material or the encapsulated nonabsorbable material from the internal or abdominal viscera or tissue and organs for a period of time after implantation. Additionally, the second absorbable material may function as an adhesion barrier to prevent postoperative adhesions between the nonabsorbable material and the internal or abdominal viscera. The second material may have a faster absorption rate than the absorption rate of the first absorbable material or component. One or more second absorbable material may be used in the repair device.

Examples of the second absorbable material include, but are not limited to, oxidized regenerated cellulose fabric such as Interceed® (TC7) absorbable adhesion barrier, gelatin films such as Gelfilm® absorbable film, and polymers or copolymers of organic hydroxyesters, polyglycolide, polylactide, polydioxanone, polyhydroxy butyric acid, polycaprolactone, polytrimethylene carbonate and polyvinyl alcohol.

In the disclosed example that is outside the scope of the claimed invention, the nonabsorbable material may function to permit the anchoring of the prosthetic repair device to the peritoneum or abdominal wall. Specifically, the nonabsorbable material may function by allowing tissue infiltration to incorporate into the prosthesis after implantation. Preferably, the nonabsorbable material is porous, such as an open cell foam, non-woven or woven structures including but not limited to a fabric, a mesh, a knit, a weave or a carded web, or porous membranes.

In the inventive embodiment, the nonabsorbable porous material provides strength to the prosthetic repair device. In addition, the nonabsorbable porous material may function to permit the anchoring of the prosthetic repair device to the peritoneum or abdominal wall after the first absorbable component has absorbed to a degree sufficient to expose the pores of the nonabsorbable material. Preferably, the nonabsorbable material is a mesh, a knit, a weave or a carded web.

The nonabsorbable material may be any biologically compatible and implantable synthetic or natural material that includes but is not limited to polyolefins such as polyethylene or polypropylene, polyesters, fluorpolymers such as polytetrafluoroethylene, polyamides such as nylon, and combinations thereof. Examples of the nonabsorbable material include but are not limited to Prolene® polypropylene mesh (commercially available from Ethicon, Inc., in Somerville, New Jersey), and Marlex® mesh.

Additionally, the prosthetic repair devices described herein may have incorporated therein one or more therapeutic agent, including but not limited to antimicrobial agents such as 2,4,4'-trichloro-2'hydroxydiphenyl ether, benzalkonium chloride, silver sulfadiazine, povidone iodine, triclosan, gentamiacin; anti-inflammatory agents, steroidal or non-steroidal, such as celecoxib, rofecoxib, aspirin, salicylic acid, acetominophen, indomethicin, sulindac, tolmetin, ketorolac, mefanamic acid, ibuprofen, naproxen, phenylbutazone, sulfinpyrazone, apazone, piroxicam, anesthetic agents such as channel blocking agents, lidocaine, bupivacaine, mepivacaine, procaine, chloroprocaine, ropivacaine, tetracaine, prilocaine, levobupivicaine, and combinations of local anesthetics with epinephrine etc., anti-proliferatives such as rapamycin, growth factors such as PGDF, scar treatment agents such as hylauronic acid, angio-genesis promoting agents, pro-coagulation factors, anti-coagulation factors, chemotactic agents, agents to promote apoptosis, immunomodulators, mitogenic agents, diphenhydramine, chlorpheniramine, pyrilamine, promethazin, meclizine, terfenadine, astemizole, fexofenidine, loratidine, aurothioglucose, auranofin, Cortisol (hydrocortisone), cortisone, fludrocortisone, prednisone, prednisolone, 6α-methylprednisone, triamcinolone, betamethasone, and dexamethasone; hemostatic agents such as thrombin, tranexamic acid, epinephrine; as well as antiviral and antithrombotic agents.

The first disclosed example of the prosthetic repair device, which is outside the scope of the claimed invention, may be made by joining the nonabsorbable material, the first absorbable material and the second absorbable material by conventional means such as stitching, tacking, lamination, compression heating, laser welding, sonic welding or via the use of an adhesive.

The prosthetic repair device of the inventive embodiment may be made, for example, by contacting a first side of the nonabsorbable material with a first film of the first absorbable component and heating the nonabsorbable material and the first absorbable component so that a portion of the nonabsorbable material is adhered to the first absorbable component. Additionally, a first side of the second absorbable material is contacted with a second film of the first absorbable component and the second absorbable material and the second film of the first absorbable component are heated so that a portion of the second absorbable material is adhered to the second film of the first absorbable component. Then the second side of the nonabsorbable material is contacted with the free side of second film of the first absorbable component and heated so that the films of the first absorbable component on both side of the nonabsorbable material melt and encapsulate the fibers and interstices of the nonabsorbable material.

Alternatively, the inventive embodiment of the repair device may be made, for example, by contacting a first side of the nonabsorbable material with a first film of the first absorbable component, and heating the nonabsorbable material with the first film of the first absorbable component so that a portion of the nonabsorbable material is adhered to the first film. Additionally, a first side of the second absorbable material is contacted with the second side of the first film of the first absorbable component which is already attached to the nonabsorbable material and the second absorbable material and the second side of the first film of the first absorbable component are heated so that a portion of the second absorbable material is adhered to the second side of the first film. Then the second side of the nonabsorbable material is contacted with a second film of the first absorbable component and heated so that the films of first absorbable component on both side of the nonabsorbable material melt and encapsulate the fibers and interstices of the nonabsorbable material.

In another alternative, the repair device may be made, for example, by contacting a first side of the nonabsorbable material with a first film of first absorbable component, and heating the nonabsorbable material and the first film so that a portion of the nonabsorbable material is adhered to the first film. Then the second side of the nonabsorbable material is contacted with a second film of the first absorbable component and heated so that the films of first absorbable component on both side of the nonabsorbable material melt and encapsulate the fibers and interstices of the nonabsorbable material. Additionally, a first side of the second absorbable material is contacted with the second side of the first film of the first absorbable component which is already attached to the nonabsorbable material and the second absorbable component and the second side of the first film are heated so that a portion of the second absorbable material is adhered to the second side of the first film.

The prosthetic repair devices described herein may be used for the repair of hernias and other fascial deficiencies. The techniques used for the repair of a hernia vary considerably. For example, the hernia repair device may be placed intraperitoneally, either via open or laparoscopic surgery. Allternatively, some surgeons prefer to place the hernia repair device extraperitoneally below or under the rectus muscle, via open or laparoscopic surgery. Optionally, the hernia repair device may be used to repair a hernia or fascial defect using an onlay technique, where the device is placed above or on top of the rectus muscle, or a subfascial technique.

### EXAMPLE

A hernia repair device was prepared utilizing a lamination system having a metal roller with a nominal diameter of 20.3 cm (8 inches) and a heating capability of up 170°C. The rotating speed of the metal roller was from 0.305 to 3.05 m/min (1 to 10 feet per minute). The lamination system also included a soft face polyurethane pressure roller with a durometer of 40 and a pressure loading of up to 681 N per linear metre (150 pounds per linear foot). One side of a 0.8 mil poly(1 ,4-dioxan-2-one) (PDS) film was covered with a first release paper (commercially available from Tekkote Corp., Leonia NJ 07605), while the other side of the PDS film was placed in contact with the smooth side of a Prolene® polypropylene mesh (PSM) product (commercially available from Ethicon, Inc. in Somerville, New Jersey). A second release paper was placed on the rough side of the PSM product to keep the components from sticking to the rollers of the lamination system. The first release paper/PDS/PSM/second release paper structure was placed into the lamination system with the metal roller set to a temperature of 157°C and running at 0.61 m/min (2 feet per minute), while the pressure roller was set to apply a load of 381 N per linear metre (70 pounds per linear inch) displaced across the face of the pressure roller, with the first release paper contacting the heated metal roller, which forced the PDS to migrate into the mesh. This step was repeated three times.

The second release paper was then removed from the rough side of the PSM product. The rough side of the PSM product was then placed in contact with the one side of 0.2 mil PDS film having a third release paper attached on its opposite side. The first release paper/PDS/PSM/PDS/third release paper structure was placed between the heated metal roller set to a temperature of 157°C and running at 0.61 m/min (2 feet per minute) and the pressure roller set to apply a load of 381 N per linear metre (70 pounds per linear inch) displaced across the face of the pressure roller, with the third release paper contacting the heated metal roller. This step was conducted once.

Then the first release paper was removed from the 0.8 PDS film and replaced with piece of Interceed® (TC7) oxidized regenerated cellulose (ORC) fabric. The ORC/PDS/PSM/ PDS/third release paper structure was placed into the lamination system with the third release paper placed against the heated metal roller. The heated metal roller was set to a temperature of 157°C and running at 0.61 m/min (2 feet per minute) and the pressure roller was set to apply a load of 381 N per linear metre (70 pounds per linear inch) displaced across the face of the pressure roller. This step was conducted once.

The structure was removed from the lamination system and hand rolled with a soft face polyurethane roller having a durometer of 40, on the release paper side of the structure, using only hand pressure. Immediately thereafter, the release paper was removed and the structure was hand rolled twice with the polyurethane roller, on the PDS side, using only hand pressure. The sample was then put into a storage bin for approximately 1 to 7 hours, then transferred and stored under vacuum until it was time to cut and package the hernia repair device.

## Claims

1. A prosthetic repair device comprising:
a nonabsorbable porous material (3) having a first side and a second side,
a first sheet of a first absorbable material (2) having a first absorption rate and having a first side and a second side,
a second sheet of the first absorbable material (2) and
a second absorbable material (1) having a faster absorption rate than the first absorption rate and having a first side and a second side,
where the second sheet of the first absorbable material (2) is proximate to the first side of the nonabsorbable material (3), the second side of the nonabsorbable material (3) is proximate to the first side of the first sheet of the first absorbable material (2) and the second absorbable material (1) is proximate to the second side of the first sheet of the first absorbable material (2), and
wherein the prosthetic repair device exhibits a hold time of from 5 minutes to 30 minutes, evaluated according to the 'Nold Time Test' defined in the description, on the basis of the second sheet of the first absorbable material (2).

2. The prosthetic repair device of claim 1, wherein the nonabsorbable material (3) is selected from the group consisting of polyolefins, polyesters, fluorpolymers, polyamides and combinations thereof; the first absorbable material (2) is selcted from the group consisting of polydioxanone, polymers or copolymers of organic hydroxyester, polyglycolide, polylactide, polyhydroxy butyric acid, polycaprolactone, polytrimethylene carbonate and polyvinyl alcohol; and the second absorbable material (1) is selected from the group consisting of oxidized regenerated cellulose, gelatin films, and polymers or copolymers of organic hydroxyesters, polyglycolide, polylactide, polydioxanone, polyhydroxy butyric acid, polycaprolactone, polytrimethylene carbonate and polyvinyl alcohol.

3. The prosthetic repair device of claim 2, wherein the nonabsorbable porous material (3) is a polyethylene, polypropylene, polyester, fluorpolymer or polyamide mesh encapsulated in a first absorbable component (2) selected from the group consisting of polydioxanone, polylactide, polyglycolide, and copolymer of caprolactone, and the second absorbable material (1) is oxidized regenerated cellulose.

4. The prosthetic repair device of claim 3, wherein the nonabsorbable material (3) is a polypropylene mesh, the first absorbable material (2) is a polydioxanone film and the second absorbable material (1) is an oxidized regenerated cellulose fabric.

5. The prosthetic repair device of claim 4, wherein the first absorbable material (2) comprises poly (1,4-dioxan-2-one).

6. The prosthetic repair device of any preceding claim, wherein the first absorbable material (2) has a melting point that is lower than the melting points of the non absorbable material (3) and the second absorbable material (1), and wherein the first sheet of the first absorbable material joins the nonabsorbable material to the second absorbable material.

7. The prosthetic repair device of any preceding claim, wherein the hold time ranges from 10 minutes to 20 minutes.

## Patentansprüche

1. Prothetische Reparaturvorrichtung, umfassend:
ein nicht-absorbierbares poröses Material (3) mit einer ersten Seite und einer zweiten Seite,
eine erste Schicht aus einem ersten absorbierbaren Material (2) mit einer ersten Absorptionsrate und mit einer ersten Seite und einer zweiten Seite,
eine zweite Schicht aus dem ersten absorbierbaren Material (2) und
ein zweites absorbierbares Material (1) mit einer schnelleren Absorptionsrate als die erste Absorptionsrate und mit einer ersten Seite und einer zweiten Seite,
wobei die zweite Schicht aus dem ersten absorbierbaren Material (2) benachbart zur ersten Seite des nicht-absorbierbaren Materials (3) ist, die zweite Seite des nicht-absorbierbaren Materials (3) benachbart zur ersten Seite der ersten Schicht aus dem ersten absorbierbaren Material (2) ist und das zweite absorbierbare Material (1) benachbart zur zweiten Seite der ersten Schicht aus dem ersten absorbierbaren Material (2) ist und
wobei die prothetische Reparaturvorrichtung eine Haltezeit von 5 Minuten bis 30 Minuten zeigt, bewertet gemäß dem "Haltezeittest", der in der Beschreibung definiert ist, auf der Basis der zweiten Schicht aus dem ersten absorbierbaren Material (2).

2. Prothetische Reparaturvorrichtung nach Anspruch 1, wobei das nicht-absorbierbare Material (3) ausgewählt ist aus der Gruppe, bestehend aus Polyolefinen, Polyestern, Fluorpolymeren, Polyamiden und Kombinationen davon; das erste absorbierbare Material (2) ausgewählt ist aus der Gruppe, bestehend aus Polydioxanon, Polymeren oder Copolymeren von organischem Hydroxyester, Polyglykolid, Polylactid, Polyhydroxybuttersäure, Polycaprolacton, Polytrimethylencarbonat und Polyvinylalkohol; und das zweite absorbierbare Material (1) ausgewählt ist aus der Gruppe, bestehend aus oxidierter regenerierter Cellulose, Gelatinefilmen und Polymeren oder Copolymeren von organischen Hydroxyestern, Polyglykolid, Polylactid, Polydioxanon, Polyhydroxybuttersäure, Polycaprolacton, Polytrimethylencarbonat und Polyvinylalkohol.

3. Prothetische Reparaturvorrichtung nach Anspruch 2, wobei das nicht-absorbierbare poröse Material (3) ein Polyethylen-, Polypropylen-, Polyester-, Fluorpolymer- oder Polyamid-Netz ist, das in einer ersten absorbierbaren Komponente (3) eingekapselt ist, die ausgewählt ist aus der Gruppe, bestehend aus Polydioxanon, Polylactid, Polyglykolid und Copolymer von Caprolacton, und das zweite absorbierbare Material (1) oxidierte regenerierte Cellulose ist.

4. Prothetische Reparaturvorrichtung nach Anspruch 3, wobei das nicht-absorbierbare Material (3) ein Polypropylen-Netz ist, das erste absorbierbare Material (2) ein Polydioxanon-Film ist und das zweite absorbierbare Material (1) ein oxidiertes regeneriertes Cellulosegewebe ist.

5. Prothetische Reparaturvorrichtung nach Anspruch 4, wobei das erste absorbierbare Material (2) Poly(1,4-dioxan-2-on) umfasst.

6. Prothetische Reparaturvorrichtung nach einem vorangehenden Anspruch, wobei das erste absorbierbare Material (2) einen Schmelzpunkt besitzt, der niedriger ist als die Schmelzpunkte des nicht-absorbierbaren Materials (3) und des zweiten absorbierbaren Materials (1), und wobei die erste Schicht aus dem ersten absorbierbaren Material das nicht-absorbierbare Material mit dem zweiten absorbierbaren Material verbindet.

7. Prothetische Reparaturvorrichtung nach einem vorangehenden Anspruch, wobei die Haltezeit von 10 Minuten bis 20 Minuten reicht.

## Revendications

1. Dispositif de réparation prothétique comprenant :
➢ un matériau poreux non absorbable (3) comportant un premier côté et un second côté,
➢ une première feuille d'un premier matériau absorbable (2) ayant une première vitesse d'absorption et comportant un premier côté et un second côté,
➢ une seconde feuille du premier matériau absorbable (2) et
➢ un second matériau absorbable (1) ayant une vitesse d'absorption plus rapide que la première vitesse d'absorption et comportant un premier côté et un second côté,
➢ ou la seconde feuille du premier matériau absorbable (2) est à proximité du premier côté du matériau non absorbable (3), le second côté du matériau non absorbable (3) est à proximité du premier côté de la première feuille du premier matériau absorbable (2) et le second matériau absorbable (1) est à proximité du second côté de la première feuille du premier matériau absorbable (2), et
> où le dispositif de réparation prothétique présente un temps de rétention de 5 minutes à 30 minutes, évalué selon "l'essai de temps de rétention" défini dans la description, sur la base de la seconde feuille du premier matériau absorbable (2).

2. Dispositif de réparation prothétique selon la revendication 1, dans lequel le matériau non absorbable (3) est choisi dans le groupe consistant en les poly(oléfines), les poly(esters), les fluoropolymères, les poly(amides) et leurs combinaisons ; le premier matériau absorbable (2) est choisi dans le groupe consistant en la poly-(dioxanone), les polymères ou copolymères d'hydroxyester organique, le poly(glycolide), le poly-(lactide), le poly(acide hydroxybutyrique), la poly(caprolactone) ; le poly(carbonate de triméthylène) et le poly (alcool vinylique) ; et le second matériau absorbable (1) set choisi dans le groupe consistant en la cellulose régénérée oxydée, les films de gélatine, et les polymères ou copolymères d'hydroxyesters organiques, le poly(glycolide), le poly(lactide), la poly-(dioxanone), le poly(acide hydroxybutyrique), la poly(caprolactone) ; le poly(carbonate de triméthylène) et le poly(alcool vinylique).

3. Dispositif de réparation prothétique selon la revendication 2, dans lequel le matériau poreux non absorbable (3) est une maille de poly-(éthylène), poly(propylène), poly(ester), fluoropolymère ou poly(amide) encapsulée dans un premier composant absorbable (2) choisi dans le groupe consistant en la poly(dioxanone), le poly-(lactide), le poly(glycolide) et un copolymère de caprolactone, et le second matériau absorbable (1) est la cellulose régénérée oxydée.

4. Dispositif de réparation prothétique selon la revendication 3, dans lequel le matériau non absorbable (3) est une maille de poly(propylène), le premier matériau absorbable (2) est un film de poly(dioxanone) et le second matériau absorbable (1) est un tissu de cellulose régénérée oxydée.

5. Dispositif de réparation prothétique selon la revendication 4, dans lequel le premier matériau absorbable (2) comprend de la poly (1,4-dioxan-2-one) .

6. Dispositif de réparation prothétique selon l'une quelconque des revendications précédentes, dans lequel le premier matériau absorbable (2) a un point de fusion qui est inférieur aux points de fusion du matériau non absorbable (3) et du second matériau absorbable (1), et où la première feuille du premier matériau absorbable joint le matériau non absorbable au secondmatériau absorbable.

7. Dispositif de réparation prothétique selon l'une quelconque des revendications précédentes, dans lequel le temps de rétention va de 10 minutes à 20 minutes.
